(19) Europäisches Patentamt
European Patent Office
Office européen des brevets

(11) Veröffentlichungsnummer: **0 039 812**
**B1**

(12) # EUROPÄISCHE PATENTSCHRIFT

(45) Veröffentlichungstag der Patentschrift: **20.07.83**

(51) Int. Cl.³: **C 07 C 51/367**, C 07 C 65/03

(21) Anmeldenummer: **81103128.5**

(22) Anmeldetag: **27.04.81**

(54) **Verfahren zur Herstellung von 3-Hydroxybenzoesäure.**

(30) Priorität: **10.05.80 DE 3017983**

(43) Veröffentlichungstag der Anmeldung: **18.11.81 Patentblatt 81/46**

(45) Bekanntmachung des Hinweises auf die Patenterteilung: **20.07.83 Patentblatt 83/29**

(84) Benannte Vertragsstaaten: **AT CH DE FR GB IT LI**

(56) Entgegenhaltungen:
**DE-A-1 065 425**
**FR-A-1 255 133**
**US-A-2 439 375**

(73) Patentinhaber: **BAYER AG, Zentralbereich Patente, Marken und Lizenzen, D-5090 Leverkusen 1, Bayerwerk (DE)**

(72) Erfinder: **Potthast, Ruthard, Dr., Martin-Buber-Strasse 45, D-5090 Leverkusen 3 (DE)**
Erfinder: **Mentzel, Werner, Dr., Morgengraben 5, D-5000 Köln 80 (DE)**
Erfinder: **Kramer, Horst-Dieter, Dr., Gabriele-Muenter-Strasse 6, D-5090 Leverkusen 1 (DE)**

**EP 0 039 812 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 3-Hydroxybenzoesäure

Die vorliegende Erfindung betrifft ein Verfahren zur Herstellung von 3-Hydroxybenzoesäure aus Sulfophthalsäure.

Aus der US 3 094 558 ist bekannt, 3-Hydroxybenzoesäure aus 3-Sulfobenzoesäure in einer Schmelze mit Kaliumhydroxid herzustellen. Die 3-Sulfobenzoesäure wird durch Sulfonierung von Benzoesäure mit Oleum oder Schwefeltrioxid hergestellt (US 3 094 558, Zeilen 20 bis 22). Bei dieser in der Technik üblichen Sulfonierung von Benzoesäure entstehen neben der 3-Sulfobenzoesäure als Hauptprodukt noch die isomeren Sulfobenzoesäuren als Nebenprodukte [Ullmann, Band 8, Zeile 380, 4. Auflage (1974)]. Die als Nebenprodukte anfallenden 2- und 4-Sulfobenzoesäuren lassen sich nicht völlig abtrennen.

Das hat zur Folge, dass man durch die Kaliumhydroxid-Schmelze keine reine 3-Hydroxybenzoesäure erhält, sondern stets ein Gemisch der isomeren Hydroxybenzoesäuren. Hinzu kommt noch, dass die 4-Hydroxybenzoesäure in der Kaliumhydroxidschmelze leicht decarboxyliert, so dass noch Phenol als weiteres Nebenprodukt bei der technischen Herstellung von 3-Hydroxybenzoesäure anfällt.

Die genannten Nebenprodukte der 3-Hydroxybenzoesäure können nur ausserordentlich schwer abgetrennt werden und beeinträchtigen eine spätere Verwendung der 3-Hydroxybenzoesäure. Sie ist ein Zwischenprodukt bei der Herstellung von Kunststoffen und Pflanzenschutzmitteln und dient als solche oder in Form ihrer einfachen aliphatischen Ester als Weichmacher.

Es wurde ein Verfahren zur Herstellung von 3-Hydroxybenzoesäure gefunden, das dadurch gekennzeichnet ist, dass man 3-Sulfophthalsäure und/oder 4-Sulfophthalsäure mit Alkalihydroxyd im Temperaturbereich von 200 bis 300°C und im Druckbereich von 10 bis 30 bar umsetzt.

Die so erhaltene 3-Hydroxybenzoesäure wird in grossen Ausbeuten und frei von störenden Nebenprodukten erhalten.

Das erfindungsgemässe Verfahren kann anhand der folgenden Reaktionsgleichung erläutert werden:

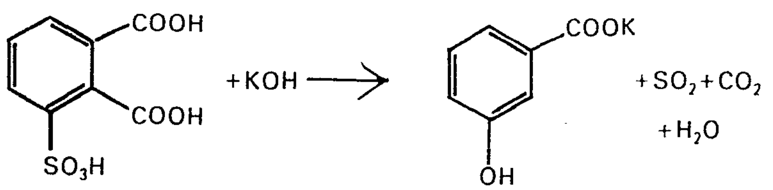


Sulfophthalsäure im Rahmen des erfindungsgemässen Verfahrens sind die 3-Sulfophthalsäure und die 4-Sulfophthalsäure. Sie können sowohl alleine als auch im Gemisch erfindungsgemäss zu der 3-Hydroxybenzoesäure umgesetzt werden. Bevorzugt verwendet man für das erfindungsgemässe Verfahren ein Gemisch der beiden isomeren Sulfophthalsäuren, wie es bei der Sulfierung von Phthalsäureanhydrid mit Oleum anfällt. Hierbei erhält man ein Gemisch von 3-Sulfophthalsäure und 4-Sulfophthalsäure im Verhältnis 1:4 bis 1:6.

Selbstverständlich ist es auch möglich, Salze der Sulfophthalsäure in das erfindungsgemässe Verfahren einzusetzen. Im allgemeinen ist es dann zweckmässig, die Alkalisalze, beispielsweise das Natrium- und/oder Kaliumsalz der Sulfophthalsäure, einzusetzen.

Als Alkalihydroxide für das erfindungsgemässe Verfahren werden im allgemeinen das Natriumhydroxid und das Kaliumhydroxid verwandt. Es ist selbstverständlich auch möglich, ein Gemisch von Natriumhydroxid und Kaliumhydroxid einzusetzen.

Für das erfindungsgemässe Verfahren setzt man im allgemeinen 7 bis 18 Mol Alkalihydroxid, bezogen auf 1 Mol der Sulfophthalsäure ein. Bevorzugt verwendet man 10 bis 12 Mol Alkalihydroxid, bezogen auf 1 Mol der Sulfophthalsäure.

Das erfindungsgemässe Verfahren wird im Temperaturbereich von 200 bis 300°C und im Druckbereich von 10 bis 30 bar durchgeführt. Bevorzugt führt man das erfindungsgemässe Verfahren im Temperaturbereich von 210 bis 280°C durch. Bevorzugter Druckbereich für das erfindungsgemässe Verfahren ist 18 bis 25 bar.

Die Sulfophthalsäuren können in das erfindungsgemässe Verfahren in Form ihrer wässrigen Lösungen eingesetzt werden. Bei dieser bevorzugten Ausführungsform des erfindungsgemässen Verfahrens werden im allgemeinen wässrige Lösungen der Sulfophthalsäure mit einem Gehalt von 30 bis 75% eingesetzt.

Das erfindungsgemässe Verfahren kann beispielsweise wie folgt durchgeführt werden:

In einem Autoklaven legt man eine wässrige Lösung des Alkalihydroxids vor und trägt in die Lösung ein Gemisch aus 3-Sulfophthalsäure und 4-Sulfophthalsäure ein. Das Reaktionsgemisch wird dann erfindungsgemäss umgesetzt. Im allgemeinen benötigt man für die Umsetzung 8 bis 15 Stunden.

Nach Beendigung der Reaktion wird die Schmelze abgekühlt und mit Wasser aufgenommen. Zur Herstellung der freien 3-Hydroxybenzoesäure säuert man das Reaktionsgemisch mit einer Mineralsäure, beispielsweise Salzsäure, an.

Auf diese Weise erhält man 3-Hydroxybenzoesäure in praktisch quantitativer Ausbeute mit grosser Reinheit. 3-Hydroxybenzoesäure wird z.B. durch Umsetzung mit Acylchloriden (4–8 C-Atome) unter katalytischem Einfluss von

Zinkchlorid zu hochmolekularen Polyestern umgesetzt [Literatur: Caldwell, US-Pat. 2.600.376 (1952)].

Die Herstellung von Pflanzenschutzmitteln erfolgt durch Nitrierung von 3-Hydroxybenzoesäure zu 2-Nitro-5-hydroxybenzoesäure und anschliessende Kondensation mit einer geeigneten Komponente zu 4-Nitro-2'-chlor-4'-trifluormethyldiphenylethercarbonsäure-(3) [Literatur: Beyer, Rec.trav.chim. 40, 613–23 (1921), C.A. 16, 1230 (1922), US-Pat. 4031 131 (1977)].

Beispiele

Beispiel 1

403,9 g (Gehalt 91,4%) eines Gemisches aus 3-Sulfophthalsäure und 4-Sulfophthalsäure in Form ihres Mono-Natriumsalzes (entspr. 369 g 100% ≙ 1,5 Mol) werden allmählich zu 960 g vorgelegter 50%iger Natronlauge (12 Mol NaOH) gegeben. Das Gesamtvolumen beträgt ca. 860 ml.

Dabei erwärmt sich die Mischung auf ca. 95°C, der 1,3 l fassende Nickel-Autoklav wird verschlossen und für die Dauer von 12 Stunden auf 235°C erhitzt. Der sich einstellende Druck beträgt 17,5 bis 18,5 bar. Nach der Reaktionszeit wird die Schmelze abgekühlt, entleert und mit 1000 ml Wasser nachgespült.

Die erhaltene farblose Kristallsuspension wird auf 80°C erwärmt und innerhalb 1 Stunde in ein Gemisch aus 1500 ml roher konzentrierter Salzsäure und 300 g Eis unter Rühren vorsichtig eingetragen. Dabei beobachtet man starke Gasentwicklung durch freiwerdendes Kohlendioxid und Schwefeldioxid. Der pH-Wert der Suspension beträgt nach beendetem Eintragen 0,7, die Temperatur der Suspension 90°C. Man treibt bei einer Temperatur um 90°C das restliche Schwefeldioxid aus und kühlt anschliessend auf 20°C ab. Für die Dauer von 3 Stunden wird nachgerührt.

Der ausgefallene, völlig farblose Niederschlag wird abgesaugt und anschliessend mit 6 Portionen von je 100 ml Wasser gewaschen.

Dann wird das erhaltene Produkt im Vakuum-Trockenschrank bei 90°C und einem Druck von 20 Torr getrocknet. Die Ausbeute beträgt 198,1 g (95,7% der Theorie) an 3-Hydroxybenzoesäure mit folgender nach Hochdruckflüssigkeitschromatografie ermittelter Analyse:

| | |
|---|---|
| 3-Hydroxybenzoesäure | 99,9% |
| 2-Hydroxybenzoesäure | nicht nachweisbar |
| 4-Hydroxybenzoesäure | nicht nachweisbar |
| Phenol | nicht nachweisbar |
| Wasser | 0,01% |
| NaCl | 0,1% |

Beispiel 2

403,9 g (91,4%) entsprechend 369 g (100%) (1,5 Mol) des trockenen Mononatriumsalzes eines Gemisches der 3-Sulfophthalsäure und der 4-Sulfophthalsäure werden in einem 1,3 l fassenden Nickel-Autoklaven zu 960 g vorgelegter 50%iger Natronlauge (12 Mol NaOH) gegeben. Der Autoklav wird verschlossen und für die Dauer von 10 Stunden auf einer Temperatur von 260°C gehalten, wobei sich ein maximaler Druck von 28 bar einstellt. Nach beendeter Reaktion lässt man den Autoklaveninhalt erkalten, entnimmt die Suspension und spült den Reaktor mit ca. 1000 ml Wasser nach. Die Suspension wird erwärmt auf 80°C und innerhalb 1 Stunde unter starkem Rühren in ein Gemisch von 1500 ml roher konzentrierter Salzsäure und 300 g Eis vorsichtig eingetragen. Dabei beobachtet man eine starke Gasentwicklung durch freiwerdendes Kohlendioxid und Schwefeldioxid. Man vertreibt nach beendetem Zulauf Reste des Schwefeldioxids durch zweistündiges Erwärmen auf 90°C und kühlt dann langsam auf Raumtemperatur ab. Der abgenutschte Kristallbrei wird im Vakuumtrockenschrank bis 90°C und 20 Torr getrocknet und ergibt eine Ausbeute von 201 g (97% der Theorie) an 3-Hydroxybenzoesäure mit einem Gehalt von 99,9%. Der Wassergehalt beträgt 0,01%, der Gehalt an Natriumchlorid 0,1%. Weitere Verunreinigungen sind nicht nachweisbar.

Beispiel 3

246,2 g (1 Mol) eines Gemisches der 3-Sulfophthalsäure und der 4-Sulfophthalsäure werden als freie Säure in Form ihrer 50%igen wässrigen Lösung (Dichte 1,295 bei 20°C) in einem Nickelautoklaven von 3 l Inhalt zu 1440 g einer 50%igen Lösung von Natriumhydroxid (720 g 100% = 18 Mol NaOH) gegeben und für die Dauer von 12 Stunden bei einer Temperatur von 260°C umgesetzt. Der sich dabei einstellende Druck beträgt 23 bar. Nach beendeter Reaktionsdauer lässt man den Autoklaven erkalten und entnimmt den Inhalt. Man spült mit 700 ml Wasser nach und erwärmt die hellgraue Suspension auf eine Temperatur von 80°C und fügt sie dann vorsichtig unter Rühren zu einem vorgelegten Gemisch von 950 ml 30%iger Salzsäure und 400 g Eis. Wobei sehr starke Kohlendioxid und Schwefeldioxidentwicklung stattfindet. Die abgekühlte Suspension wird bei Raumtemperatur auf einer Nutsche getrennt und der farblose Kristallkuchen im Vakuumtrockenschrank bei 90°C und 20 Torr 10 Stunden getrocknet. Die Ausbeute an 3-Hydroxybenzoesäure beträgt 131 g (95% der Theorie) Gehalt an 3-Hydroxybenzoesäure 99,9%.

Beispiel 4

(Vergleichsbeispiel: Schmelze von 3-Sulfobenzoesäure zu 3-Hydroxybenzoesäure)

404,4 g feuchtes Mononatriumsalz der 3-Sulfobenzoesäure (Gehalt 70%), entspricht 313,9 g Mononatriumsalz = 283,1 g freie Säure MG 202,2 = 1,4 M (erhalten durch Sulfierung von Benzoesäure) werden zu 500 ml einer 50%igen Lösung von Natriumhydroxyd (760 g effektiv, 380 g 100% = 9,5 M), die in einem 3-l-Nickelautoklaven vorgelegt ist, gegeben.

Danach wird der Autoklav geschlossen, auf 280°C geheizt und für die Dauer von 6 Stunden bei dieser Temperatur gehalten.

Nach vollständigem Umsatz wird die Schmelze innerhalb einer Stunde durch vorsichtige Zugabe

von 500 ml Wasser abgekühlt und verdünnt. Die verdünnte Schmelzlösung gibt man allmählich in 1,5 l wässrige Salzsäure (d = 1,151). Anschliessend wird innerhalb von 3 Stunden Schwefeldioxid mit Luft ausgeblasen und die erhaltene Suspension auf ca. 15°C abgekühlt. Das Produkt wird als Feuchtware auf der Nutsche isoliert und mit ca. 500 ml Wasser gewaschen. Die Ware wird im Vakuum getrocknet.

Ausbeute: 174,0 g 3-Hydroxybenzoesäure (90% der Theorie). Zusammensetzung der eingesetzten 3-Sulfobenzoesäure:

| | |
|---|---|
| 95,5% | 3-Sulfobenzoesäure |
| 0,68% | 2-Sulfobenzoesäure |
| 3,8% | 4-Sulfobenzoesäure |

Zusammensetzung der erhaltenen 3-Hydroxybenzoesäure:

| | |
|---|---|
| 99,3% | 3-Hydroxybenzoesäure |
| 0,25% | 2-Hydroxybenzoesäure |
| 0,26% | 4-Hydroxybenzoesäure |
| 0,06% | Benzoesäure |
| 0,11% | NaCl |
| 0,03% | Wasser |

**Patentansprüche**

1. Verfahren zur Herstellung von 3-Hydroxybenzoesäure, dadurch gekennzeichnet, dass man 3-Sulfophthalsäure und/oder 4-Sulfophthalsäure mit Alkalihydroxyd im Temperaturbereich von 200 bis 300°C und im Druckbereich von 10 bis 30 bar umsetzt.
2. Verfahren nach Anspruch 1, dadurch gekennzeichnet, dass man bei Temperaturen von 210 bis 280°C arbeitet.
3. Verfahren nach Ansprüchen 1 bis 2, dadurch gekennzeichnet, dass man bei Drücken von 18 bis 25 bar arbeitet.
4. Verfahren nach Ansprüchen 1 bis 3, dadurch gekennzeichnet, dass man 7 bis 18 Mol Alkalihydroxid, bezogen auf 1 Mol der Sulfophthalsäure, einsetzt.
5. Verfahren nach Ansprüchen 1 bis 3, dadurch gekennzeichnet, dass man 10 bis 12 Mol Alkalihydroxid, bezogen auf 1 Mol der Sulfophthalsäure, einsetzt.
6. Verfahren nach Ansprüchen 1 bis 5, dadurch gekennzeichnet, dass man Sulfophthalsäure in wässriger Lösung einsetzt.
7. Verfahren nach Ansprüchen 1 bis 6, dadurch gekennzeichnet, dass man eine wässrige Lösung der Sulfophthalsäure mit einem Gehalt von 30 bis 75% einsetzt.
8. Verfahren nach Ansprüchen 1 bis 7, dadurch gekennzeichnet, dass man die Alkalisalze der Sulfophthalsäure einsetzt.

**Claims**

1. Process for the prepatation of 3-hydroxybenzoic acid, characterised in that 3-sulphophthalic acid and/or 4-sulphophthalic acid is reacted with an alkali metal hydroxide in the temperature range of 200 to 300°C and in the pressure range of 10 to 30 bars.
2. Process according to Claim 1, characterised in that the reaction is carried out at temperatures of 210 to 280°C.
3. Process according to Claims 1 to 2, characterised in that the reaction is carried out under pressures of 18 to 25 bars.
4. Process according to Claims 1 to 3, characterised in that 7 to 18 mols of alkali metal hydroxide are employed, relative to 1 mol of sulphophthalic acid.
5. Process according to Claims 1 to 3, characterised in that 10 to 12 mols of alkali metal hydroxide are employed, relative to 1 mol of sulphophthalic acid.
6. Process according to Claims 1 to 5, characterised in that sulphophthalic acid is employed in aqueous solution.
7. Process according to Claims 1 to 6, characterised in that an aqueous solution of the sulphophthalic acid with a content of 30 to 75% is employed.
8. Process according to Claims 1 to 7, characterised in that the alkali metal salts of sulphophthalic acid are employed.

**Revendications**

1. Procédé pour la fabrication d'acide 3-hydroxybenzoïque, caractérisé en ce que l'on fait réagir l'acide 3-sulfophtalique et/ou l'acide 4-sulfophtalique avec un hydroxyde alcalin dans un intervalle de températures de 200 à 300°C et un intervalle de pressions de 10 à 30 bars.
2. Procédé selon la revendication 1, caractérisé en ce que l'on opère à des températures de 210 à 280°C.
3. Procédé selon les revendications 1 à 2, caractérisé en ce que l'on opère sous des pressions de 18 à 25 bars.
4. Procédé selon les revendications 1 à 3, caractérisé en ce que l'on utilise 7 à 18 moles d'hydroxyde alcalin pour 1 mole de l'acide sulfophtalique.
5. Procédé selon les revendications 1 à 3, caractérisé en ce que l'on utilise 10 à 12 moles d'hydroxyde alcalin pour 1 mole de l'acide sulfophtalique.
6. Procédé selon les revendications 1 à 5, caractérisé en ce que l'on utilise l'acide sulfophtalique en solution aqueuse.
7. Procédé selon les revendications 1 à 6, caractérisé en ce que l'on utilise une solution aqueuse de l'acide sulfophtalique ayant une teneur de 30 à 75%.
8. Procédé selon les revendications 1 à 7, caractérisé en ce que l'on utilise les sels alcalins de l'acide sulfophtalique.